# EUROPEAN PATENT APPLICATION

(11) **EP 1 992 610 A1**
(43) Date of publication of application: **19.11.2008**
(21) Application number: 07290633.2
(22) Date of filing: 16.05.2007
(51) Int. Cl.: C07C 229/48, A61K 31/195, C07K 1/107

(54) **New route of synthesis**

(71) Applicant: Faust Pharmaceuticals, 67400 Illkirch (FR)
(72) Inventor: Schann, Stephen, 67118 Geispolsheim (FR); Mayer, Stanislas, 67114 Eschau (FR)

(57) **Abstract**

The invention relates to efficient methods of synthesis for compounds which are effectors of central nervous system receptors sensitive to neuroexcitatory amino acids such as glutamate or for synthetic precursors or intermediates of these compounds. In particular, the invention provides synthetic strategies and methods which can suitably be applied in the stereoselective synthesis of compounds included in the chemical family of aminocyclopentane tricarboxylic acids.

## Description

The invention relates to efficient methods of synthesis for compounds which are effectors of central nervous system receptors sensitive to neuroexcitatory amino acids such as glutamate or for synthetic precursors or intermediates of these compounds. In particular, the invention provides synthetic strategies and methods which can suitably be applied in the stereoselective synthesis of compounds included in the chemical family of aminocyclopentane tricarboxylic acids.

It is known that glutamate is involved in numerous cerebral functions. Important roles are therefore attributed to glutamatergic receptors, in particular as regards the conduction of nerve impulse, synaptic plasticity, the development of the central nervous system, learning and memory.

Glutamate is also the main endogenous neurotoxin, being responsible for the neuronal death observed after ischemia, hypoxia, epileptic fits or traumatisms of the brain. It is not therefore surprising that glutamate receptors are considered to be involved in various disorders of the central nervous system and neurodegenerative diseases. Two main types of glutamatergic receptors have been characterized: ionotropic receptors and metabotropic receptors. The ionotropic receptors are cationic channels activated by the glutamate and directly responsible for the rapid depolarization of post-synaptic cells. They are compounds of different sub-units and classified into three groups according to their pharmacological and functional properties. A distinction is thus made between the NMDA receptors (N-methyl-D-aspartic acid), AMPA (alpha-amino-3-hydroxy-5-methyl-isoxazole-4-propionic acid) and kainate. Metabotropic receptors (mGluRs) have been revealed in the mid eighties (1985). They have been found to play an important role in particular in the induction of the long-term potentialization (LTP) and the long-term depression (LDP) of synaptic transmission, in the regulation of baroceptive reflexes, spatial learning, motor learning, postural and kinetic integration, and are likely involved in acute or chronic degenerative diseases such as epilepsy, Alzheimer's disease, Parkinson's disease or Huntington chorea as well as neuropsychiatric disorders such as anxiety, depression and schizophrenia.
A new class of ligands of metabotropic glutamate receptors is disclosed in WO 98/43907 and the corresponding US-Patent No. 6,433,014. Among the cyclic analogues of glutamic acid disclosed in these documents, 1-aminocyclopentane-1,3,4-tricarboxylic acids (ACPTs) have been found to be of great interest. In particular, the compound identified as ACPT-1 or meso-cis ACPT ((1S, 3R, 4S) -1-aminocyclopentane-1,3,4-tricarboxylic acid) has shown to be efficient as a selective group III metabotropic glutamate receptor agonist with a high potential as an active agent for modulating the effect of glutamate.

However, the synthetic routes that have been known for the preparation of ACPTs, and in particular of pure stereoisomeric forms thereof, e.g. from WO 98/34907 suffer from limitations such as formation of several stereoisomers, challenging purification and a reduced yield of the target compounds, which make their scale-up inconvenient.

### BRIEF SUMMARY OF THE INVENTION

In view of the above, it is the aim of the invention to provide efficient methods for the synthesis of compounds of the ACPT family which provide specific stereoisomers thereof in high yields. In particular, the invention relates methods for the provision of ACPT-1 or meso-cis ACPT ((1S, 3R, 4S) -1-aminocyclopentane-1,3,4-tricarboxylic acid) and its esters or amides. To that extent, the invention includes methods which yield synthetic precursors or intermediates of the above compounds which can be conveniently converted to the desired active compounds by reactions known in the art.

The invention thus concerns a method for the preparation of a compound of formula (I): 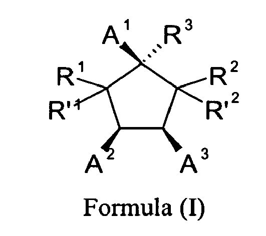 wherein
A¹, A² and A³ are independently selected from a carboxylic acid group and a salt, ester or amide thereof, from an aldehyde group and protected forms thereof, from a -COCCl₃ group and protected forms thereof, from a -COCBr₃ group and protected forms thereof, and from a -CH₂-OH group and protected forms thereof;
R¹, R'¹, R² and R'² are independently selected from hydrogen, optionally substituted alkyl, optionally substituted aryl, halogen, OH or O-alkyl;
R³ is selected from an amino group which may be mono- or disubstituted and/or which may form a salt, an isocyano group, an imino group which may be substituted, a nitro group and a -NHY group, with Y representing a -(W)ₙ-H group wherein W represents an amino-acyl group corresponding to natural or non-natural amino-acid and n represents an integer comprised between 1 and 10
and wherein A¹ and R³, together with the carbon atom to which they are attached, may form a cyclic group;
said method comprising the step of hydrogenating compound of formula (II) 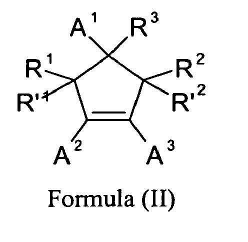 wherein A¹, A², A³, R¹, R'¹, R², R'² and R³ are defined as for formula (I), in the presence of a catalyst.

The method of the invention is easy to implement and can be exploited on an industrial scale. In particular, it has been surprisingly found that the hydrogenation reaction proceeds with a high selectivity of more than 80 or more than 90 and in particular up to 95 or 100 % to yield products of formula (I) wherein A¹, A² and A³ have the relative positions indicated in formula (I) above. Thus, a separation of different stereoisomers after the hydrogenation reaction to obtain the compounds of formula (I) pure form can be dispensed with.

According to a preferred aspect, the method according to the invention further comprises the steps of subjecting a compound of formula (III) 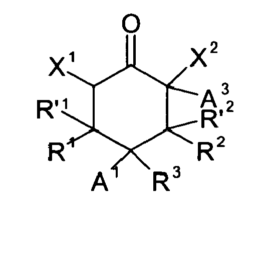 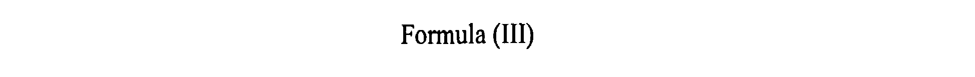 wherein
A¹, A³, R¹, R'¹, R², R'² and R³ are as defined above and X¹ and X² may be the same or different and represent each a leaving group, to a Favorskii rearrangement to yield a compound of formula (II) as defined above.

### DETAILED DESCRIPTION OF THE INVENTION

The term "alkyl" as used herein, unless indicated otherwise, refers to a respective group preferably having 1 to 20 carbon atoms, more preferably 1 to 10 and particularly preferably 1 to 8 or 1 to 6 carbon atoms. Such alkyl groups may be linear, branched or cyclic and include methyl, ethyl, propyl, butyl, pentyl and hexyl groups. An alkyl group may be unsubstituted or may carry one ore more substituents, which may be selected, e.g., from hydroxyl, halogen and aryl.
The term "aryl" as used herein, unless indicated otherwise, refers to a respective group preferably having 6 to 18 carbon atoms, particularly 6 to 10 carbon atoms. It includes groups such as a phenyl or naphtyl group. An aryl group may be unsubstituted or may carry one or more substituents, which may be selected, e.g., from alkyl, hydroxyl, halogen, O-alkyl and nitro.
The term "leaving group" as used herein, unless indicated otherwise, refers to a group of charged or uncharged atoms that departs during a substitution or displacement reaction. Also known as nucleofuge.
The term "halogen" as used herein, unless indicated otherwise, includes atoms such as fluorine, chlorine, bromine or iodine.
In the compounds of formula (I), A¹, A² and A³ may be the same or different and are independently selected from a carboxylic acid group and a salt, ester or amide thereof, from an aldehyde group and protected forms thereof, from a -COCCl₃ group and protected forms thereof, from a -COCBr₃ group and protected forms thereof, and from a -CH₂-OH group and protected forms thereof. Preferably, A² and A³ are identical. Particularly preferred is the case where A¹, A² and A³ are identical.
Carboxylic acid salts as A¹, A² and/or A³ may be formed with any inorganic or organic cation. Preferably, pharmaceutically acceptable cations are used. However, this is not an essential requirement, since the cation may be exchanged to a pharmaceutically acceptable one during further steps of the synthetic method. Exemplary inorganic cations include alkali metal cations such as sodium and potassium ions and ammonium ions. Exemplary organic cations include ammonium ions substituted by one, two, three or four alkyl groups.
Carboxylic acids as A¹, A² and/or A³ may be protected with protective groups known in the art (cf. e.g. Green, T;W.; Wuts, P.G.M. Protective Groups in Organic Synthesis, 3nd ed.; Wiley, New York, 1999, chapter 5, page 369).
The structure of the ester groups of a carboxylic acid as A¹, A² and/or A³ is not particularly restricted in the context of the present invention, since any ester group which may be less preferred for the intended use of the target compound may be removed or exchanged in additional steps of the synthetic method. Such ester groups preferably have the structure -COOR⁵, wherein R⁵ is an alkyl or an aryl group. Preferred for R⁵ group is an alkyl group. Particularly preferred for R⁵ is a C1-C4 alkyl group, such as methyl, ethyl, i-propyl or t-butyl.
Likewise, the structure of the amide groups of a carboxylic acid as A¹, A² and/or A³ is not particularly restricted in the context of the present invention. Such amide groups of a carboxylic acid as A¹, A² and/or A³ preferably have the structure -CONR⁶R⁷, wherein R⁶ and R⁷ are independently selected from hydrogen, an alkyl or an aryl group. Preferably, R⁶ and R⁷ represent hydrogen or an alkyl. Particularly preferably, R⁶ and R⁷ are selected from hydrogen and from a C1-C4 alkyl group, such as methyl, ethyl, i-propyl or t-butyl.
The term "protected forms of an aldehyde" as used in the definition of A¹, A² and A³ herein relates to a group -CHO which has been reacted with a protective group for the carbonyl C=O function as it is widely known in the art (cf. e.g. Green, T;W.; Wuts, P.G.M. Protective Groups in Organic Synthesis, 3nd ed.; Wiley, New York, 1999, chapter 4, page 293). Preferred examples thereof are acyclic and cyclic acetals.
The terms "protected forms of -COCCl₃" and "protected forms of -COCBr₃" as used in the definition of A¹, A² and A³ herein relates to a protection of the carbonyl C=O function as it is widely known in the art (cf. e.g. Green, T;W.; Wuts, P.G.M. Protective Groups in Organic Synthesis, 3nd ed.; Wiley, New York, 1999, chapter 4, page 293). Preferred examples thereof are acyclic and cyclic acetals.
The term "protected forms of the alcohol" as used in the definition of A¹, A² and A³ herein relates to a group -CH₂-OH which has been reacted with a protective group for the alcoholic -OH function as it is widely known in the art (cf. e.g. Green, T;W.; Wuts, P.G.M. Protective Groups in Organic Synthesis, 3nd ed.; Wiley, New York, 1999, chapter 2, page 17). Preferred examples thereof are esters, preferably those with carboxylic acids having 1 to 6 carbon atoms such as formic, acetic or propionic acid and ethers, preferably those with alcohols having 1 to 6 carbon atoms, such as methanol, ethanol, propanol or butanol.
Particularly preferred groups A¹, A² and A³ are carboxylic acid groups or esters thereof with protective groups known in the art.

In the compounds of formula (I), R¹, R'¹, R² and R'² are independently selected from hydrogen, alkyl, aryl, halogen, OH or O-alkyl. Preferred as R¹, R'¹, R² and R'² are hydrogen, halogen and non-substituted C1 to C4 alkyl. Particularly preferred are fluorine and hydrogen and more particularly preferred is hydrogen.
In the compounds of formula (I), R³ can be an amino group which can be protected by protective groups known in the art (cf. e.g. Green, T;W.; Wuts, P.G.M. Protective Groups in Organic Synthesis, 3nd ed.; Wiley, New York, 1999, chapter 7, page 494).
In the compounds of formula (I), R³ is independently selected from an amino group which may be mono- or disubstituted and/or which may form a salt, an isocyano group, an imino group which may be substituted, a nitro group, and a NHY group. The amino group which may be mono- or disubstituted is preferably represented by the formula - NR⁸R⁹, wherein R⁸ and R⁹ are independently selected from hydrogen, alkyl or aryl. Preferably, R⁸ and R⁹ represent hydrogen or an alkyl group. Particularly preferably, R⁸ and R⁹ are selected from hydrogen and from a C1-C4 alkyl group, such as methyl, ethyl, i-propyl or t-butyl and most preferred is hydrogen. A salt which is optionally formed of an amino group R³ may be a salt with an inorganic acid, such as hydrochloric or hydrobromic acid or a salt with an organic acid such as acetic acid.

The imino group which may be substituted is preferably represented by the formula -N=CR¹⁰R¹¹ wherein R¹⁰ and R¹¹ are independently selected from hydrogen, alkyl or aryl. Preferably, R¹⁰ and R¹¹ represent hydrogen or an aryl group. Particularly preferably, R¹⁰ and R¹¹ are selected from hydrogen or from phenyl and most preferably R¹⁰ and R¹¹ are phenyl.

In the group -NHY as one embodiment of R³, Y represents a -(W)ₙ-H group wherein W represents an amino-acyl group corresponding to a natural or non-natural amino-acid and n represents an integer comprised between 1 and 10.

In the group -NHY, W is an amino-acyl group -C(O)-L-NH- wherein the structure of L is defined such that the amino acyl group corresponds to a natural or non-natural amino-acid and wherein the carbonyl group of W or of the first of a chain of n groups W forms an amide bond with the amino function to which Y is bound. An amino-acid is a molecule that contains at least one amine function and one carboxylic acid function.
In cases where W is an amino-acyl group corresponding to one of the twenty natural amino-acids, such natural amino-acid could be glycine (Gly), leucine (L-Leu), phenylalanine (L-Phe), alanine (L-Ala), valine (L-Val), isoleucine (L-Ile), histidine (L-His), aspartic acid (L-Asp), glutamic acid (L-Glu), asparagine (L-Asn), glutamine (L-Gln), arginine (L-Arg), tyrosine (L-Tyr), cysteine (L-Cys), methionine (L-Met), lysine (L-Lys), serine (L-Ser), threonine (L-Thr), tryptophan (L-Trp), and proline (L-Pro).
In cases where W is an amino-acyl corresponding to a non natural amino-acid, such amino-acid could be the D-isomers of the natural amino-acids indicated above, beta-alanine (β-Ala), allylglycine, alloisoleucine (αIle), norvaline (Nva), norleucine (Nle), neopentylglycine, 2-amino-3,3-dimethylbutyric acid (tert-Leu), citrulline (Cit), cystathionine, cysteic acid, L-DOPA, homocysteine, homoserine, lanthionine, ornithine, 5-oxoproline, selenocysteine, thyronine, thyroxine, 2-aminobutyric acid (Abu), 2,4-diaminobutyric acid (A₂bu), 2-aminoisobutyric acid (β-Aib), 3-aminobutyric acid, 4-aminobutyric acid (GABA), 2-aminohexanoic acid (Ahx), 6-aminohexanoic acid (εAhx), 2-aminoadipic acid (Aad), 3-aminoadipic acid (βAad), 2,3-diaminopropionic acid (A₂pr), ornithine (Orn), 2-aminopimelic acid (Apm), 2,6-diaminopimelic acid (A₂pm), 2,3-diaminopropionic acid (Dpr), 2-aminotetradecanoic acid, 3-(2-naphtyl)alanine (2-Nal), 3-(1-naphtyl)alanine (1-Nal), β-cyclopropyl-alanine, β-cyclohexyl-alanine (Cha), 1-amino-cyclohexane acetic acid, 1-aminomethyl-cyclohexane acetic acid (Gabapentin), 3-(aminomethyl)-5-methylhexanoic acid (pregabalin), baclofen, saclofen, vigabatrin, LY354740, LY404039, ACPT-I, (+)-ACPT-III, 2-aminoisobutyric acid, 1-amino-1-cyclopropane carboxylic acid, 1-amino-1-cyclobutane carboxylic acid, 1-amino-1-cyclopentane carboxylic acid, 1-amino-1-cyclohexane carboxylic acid, hydroxyproline, sarcosine (Sar), O-methyl-tyrosine, phenyl glycine and the like; and amino-acids in which the α-carbon bears two side-chain substituents; and cyclic amino-acids.

In the group -NHY, n is an integer between 1 and 10. Preferably between 1 and 5 and more preferably n is 1 or 2 (each range including the upper and lower limit). In cases where n is an integer of 2 or more, the groups W may be identical or different and are generally linked to each other via amide bonds to form an oligopeptide structure.

Groups corresponding to R³ in the target compound may be further converted to other groups R³ as desired / necessary during the course of the reaction sequence.

Particularly preferred as R³ is an amino group NH₂ or a protected form thereof known in the art, or a NHY group as defined above.

Alternatively, A¹ and R³, together with the carbon atom to which they are attached, may form a cyclic group containing at least one N atom, such as hydantoine or 5-oxazolone or 2,5-dihydro-3,6-dimethoxy-2-isopropylpyrazine. These cyclic groups are preferably bound to the carbon atom of the cyclopentane ring in formula (I) via a nitrogen atom.

In formula (II), the variables corresponding to those of formula (I) have the same meaning as given above for formula (I), including the preferred embodiments.

The hydrogenation of the compound of formula (II) is carried out in the presence of a catalyst. During this reaction, hydrogen is added to the double bond in the cyclopentene ring indicated in formula (II). Surprisingly, is has been found that the compounds of formula (II) can be hydrogenated with a high selectivity of up to 100 % to yield products of formula (I) wherein A¹, A² and A³ have the relative positions indicated in formula (I) above. Without whishing to be bound to any theory, it is hypothesized that this selectivity is a result of an interaction between the catalyst and the nitrogen atom of R³.

Preferred catalysts are compounds comprising one or more transition metals. They may be used as heterogeneous catalysts, e.g. in the form or elementary metals or their oxides, optionally carried on supports such as active carbon or ceramics. Alternatively, transition metal complexes known as hydrogenation catalysts may be used for homogeneous catalysis of the hydrogenation reaction.

Generally preferred both for heterogeneous and homogeneous catalysts are transition metals from group 8, 9 and 10 of the periodic table of the elements. Particularly preferred are Ru, Rh, Ir, Ni, Pd and Pt. Particular examples of suitable catalysts are Pt or Pd supported on active carbon (Pt/C or Pd/C) or other forms of elementary platinum or palladium, Raney nickel, PtO2, nickel boride, rhodium, ruthenium, Wilkinson's catalyst, Crabtree's catalyst, among which further preference is given to PtO₂ and Pd/C. In order for the hydrogenation reaction to proceed with excellent selectivity, conventional hydrogenation catalysts can be used which do not need to provide any kind of sterical hindrance or chirality.

Hydrogen as a reactant may be used in the form of hydrogen gas. Alternative sources of hydrogen include hydrazine, dihydronaphtalene, dihydroanthracene, cyclohexene, isopropanol or formic acid. The hydrogenation reaction can be carried out with the compound of formula (II) being present in a solvent, such as an alcohol, including methanol or ethanol, ethyl acetate, acetic acid, dimethyl-formamide or water. Alternatively, an acid such as hydrochloric acid or acetic acid could be added to the solution to form the salt of the amine.

The hydrogen pressure during the reaction typically varies between 1 to 100 atm, preferably from 1 to 10 atm.

The temperature during the hydrogenation reaction typically varies between 0°C and 150°C, preferably between 0°C and 100°C, more preferably between 20°C and 50°C.

One selected set of conditions for the hydrogenation of a compound of formula (II) to yield a compound of formula (I) would be the hydrogenation using PtO₂ in the form of a powder in ethanol as a solvent at room temperature and hydrogen gas at a pressure of 1 atm as a reactant.

In order to obtain a compound of formula (I) wherein one, two or all of the groups A¹, A² and A³ are represented by a carboxylic acid group -COOH, the respective group(s) is (are) preferably protected by a protecting group before carrying out the hydrogenation. Alternatively, if a reactant of formula (II) is used wherein the respective groups A¹, A² or A³ represent -CH₂-OH or an aldehyde, protected or not, the alcohol or the aldehyde group can be oxidized to yield a carboxylic group after the hydrogenation of the double bond indicated in the cyclopentene ring in formula (II). Similarly, if a reactant of formula (II) is used wherein the respective groups A¹, A² or A³ represent - COCCl₃ or -COCBr₃, protected or not, those ketones can be cleaved to yield a carboxylic group or an ester group after the hydrogenation of the double bond indicated in the cyclopentene ring in formula (II).

If one ore more protected carboxylic acid groups are present in the compounds of formula (I) during the hydrogenation reaction, the method according to the invention may further comprise the step of deprotecting the carboxylic acid groups in the compounds of formula (I) after the hydrogenation reaction to yield compounds of formula (I) wherein the carboxylic acid groups are present in their free form. The deprotection can be effected according to methods established in the art (cf. e.g. Green, T;W.; Wuts, P.G.M. Protective Groups in Organic Synthesis, 3nd ed.; Wiley, New York, 1999, chapter 5, page 373). For example, alkyl ester groups as protective groups may be removed via addition of an acid, e.g. hydrochloric acid, sulphuric acid, hydrobromic acid or of trimethylsilyl iodide, or of a base such as LiOH.

Various synthetic routes are available in order to provide the reactants of formula (II). For example, they may be conveniently obtained by subjecting a compound of formula (III) 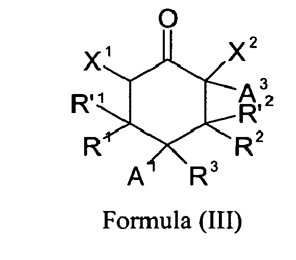 wherein
A¹, A³, R¹, R'¹, R², R'² and R³ are as defined above in formula (I) and X¹ and X² may be the same or different and represent each a leaving group, to a Favorskii rearrangement, to yield a compound of formula (II).

Thus, in one embodiment of the invention, the hydrogenation of a compound of formula (II) to yield a compound of formula (I) is preceded by a method comprising the above reaction steps to convert a compound of formula (III) into a compound of formula (II). In formula (III), the variables corresponding to those of formula (I) have the same meaning as given above for formula (I), including the preferred embodiments.

X¹ and X² represent leaving groups known in the art. Preferred groups for this purpose are halogen atoms, such as chlorine, bromine or iodine or mesylate, triflate or tosylate groups. In a particularly preferred embodiment, X¹ and X² are identical and both represent chlorine or bromine, in particular bromine.

Starting from a compound of formula (III), the Favorskii rearrangement is effected via addition of a base, e.g. an alkali metal salt of a C1-C4 alkyl-OH, such as sodium ethanolate or potassium ethanolate. During this rearrangement the six member ring was modified to a five member ring system and the keto group was oxidized to a A² group (cf. e.g. March, J; Smith, M.B. March's Advanced Organic Chemistry, 5th ed.; Wiley, New York, 2001, page 1403). Further elimination of HX¹, which is also assisted by the added base, yields the compound of formula (II), wherein A² is a carboxylic acid group or its ester or its salt, respectively. The group A² can subsequently be converted to other groups A², as required. During the conversion of the compound of formula (III) to yield a compound of formula (II), R³ is preferably a protected amino group, e.g. an amino group converted to a substituted imino group -N=CR¹⁰R¹¹as defined for the compounds of formula (I). The protective group may be removed after the Favorskii rearrangement, e.g. via addition of an acid such as hydrochloric acid or citric acid. Suitable conditions for the removal of a protective group will be readily chosen by the skilled person based on common general knowledge.

The reaction steps to convert the compound of formula (III) into a compound of formula (II) are preferably carried out in a polar solvent, such as methanol, ethanol or THF. The reaction may be suitably carried out in a temperature range of -78°C to 150°C. Preferred are temperatures between -20 °C to room temperature.

The compounds of formula (III), in turn, can be obtained by introducing the leaving groups X¹ and X² to a compound of formula (IV): 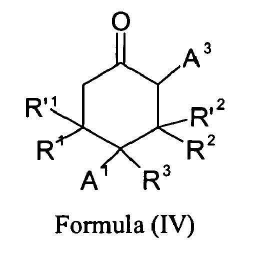 wherein A¹, A³, R¹, R'¹, R², R'² and R³ are defined as in the case of the compounds of formula (I).
For example, in cases where X¹ and X² are halogens, the compound of formula (IV) may be converted to a compound of formula (III) by reaction with halogenating agents such as Cl₂ or Br₂. Alternatively, N-bromo-succinimide or N-chloro-succinimide can be used as halogenating agents. The halogenating agent may be conveniently reacted with a solution of the compound of formula (IV), e.g. in CHCl₃, ClCH₂CH₂Cl, CCl₄, CH₂Cl₂, acetic acid, ethyl acetate or other solvents. For example, a bromination reaction may be carried out, e.g., by adding the bromine to the solution of the compound of formula (IV) at a temperature ranging from -5 °C to room temperature.

Finally, compounds of formula (IV) can be obtained, e.g., by a reaction of compounds of formula (V) below with compounds of formula (VI) below, wherein A¹, A³ R¹, R'¹, R², R'² and R³ are defined as in the case of the compounds of formula (I), according to the type of reaction described in A. Lopez et al., Tetrahedron, 52(24), 1996, 8365-8386*.* In this reaction, at least 2 moles of compound (VI) are reacted per mole of compound of formula (V) in the presence of a base. The reaction may be conveniently carried out in the presence of a solvent, such as acetonitrile, under solid-liquid phase transfer catalysis as described in the above publication. As a phase transfer catalyst, Bu₄NBr or other compounds known for this purpose may be used. Alternatively, this reaction can be performed in the absence of transfer catalyst. 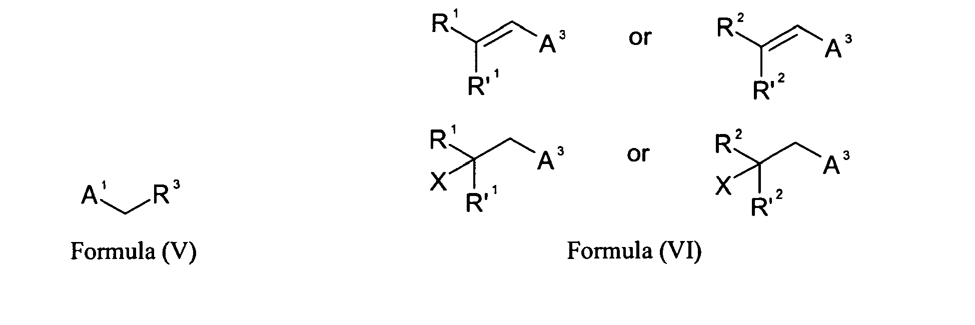 A general synthetic strategy which can be contemplated to obtain compounds of formula (IV) reacts compounds which are considered as glycine synthons (corresponding to formula (V) and such as the ones depicted below) with compounds which are considered as propionate synthons (corresponding to formula (VI) and such as the ones depicted below), 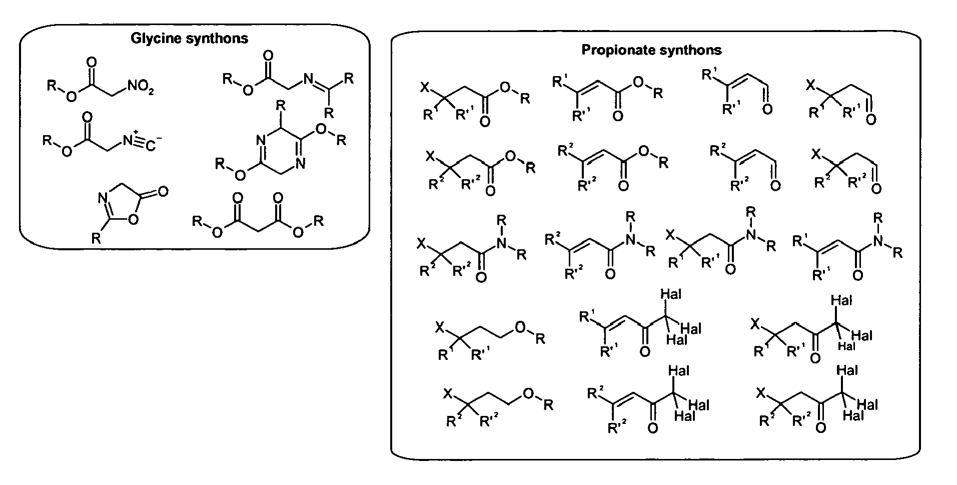 wherein R represents hydrogen, alkyl or aryl, preferably hydrogen or alkyl, X represents a leaving group, such as bromine, chlorine, iodine, mesylate, triflate or tosylate, Hal represents a bromine or a chlorine and R¹, R'¹, R² and R'² are as defined for formula (I).

A particularly preferred reaction sequence in order to obtain a preferred compound of formula (I) (here indicated as ACPT-I) is shown in the following scheme:

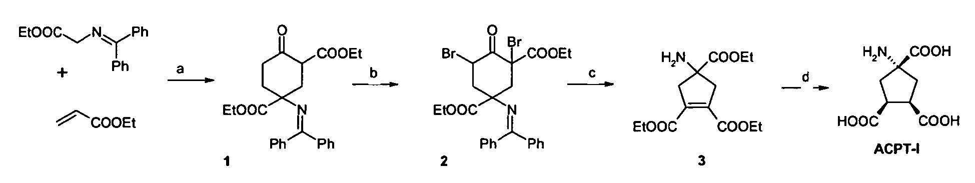

In step (a), ring formation is effected in the presence of a base to yield intermediate 1 (a preferred compound of formula (IV)). In step (b), compound 1 is brominated to yield intermediate 2 (a preferred compound of formula (III)). In step (c), the Favorskii rearrangement forms a compound having a cyclopentene skeleton, and the deprotection of the amino group yields to intermediate 3 (a preferred compound of formula (II)). This intermediate is hydrogenated in step (d) in the presence of a catalyst, and the ester groups are removed to yield the target compound ACPT-I, a preferred compound of formula (I).

Of course, based on common general knowledge the skilled person can choose a variety of other synthetic routes than the one described above to obtain compounds embraced by formula (II) to be subjected to the hydrogenation reaction in accordance with the invention. As non-limiting examples, a number of possible methods shall be set out in the following.

As a first example, selected compounds of formula (II) can be obtained starting from dimethylmaleic acid anhydride or dimethylmaleic amide, functionalizing the methyl substituents with a suitable leaving group X and reacting the resulting compound with a glycine synthon as illustrated above in accordance with the following reaction scheme. Groups corresponding to A¹ to A³ or R¹ to R³ or R'¹ to R'² in the target compound may be further converted to other groups A¹ to A³ or R¹ to R³ or R'¹ to R'² as desired/necessary during the course of the reaction sequence:

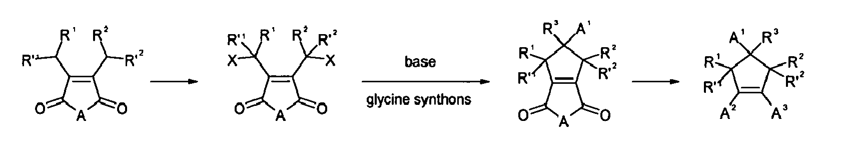

In the formulae, A is oxygen or NRi, Ri represents hydrogen, alkyl or aryl, preferably alkyl or aryl, X represents a leaving group, such as bromine, chlorine, iodine, mesylate, triflate or tosylate and R¹, R², R³, R'¹, R'², A¹, A² and A³ are as defined for formula (I). Examples of reactions used in such a synthetic sequence are given in the two following articles: Synthesis, 8, 2002, 1010-1012 and Tetrahedron Letters, 44, 2003, 9213-9217 describing bromination of dimethylmaleic anhydride for the first one and di-alkylation of a bis(allylic bromide) derivative for the second one.

A further alternative approach for the synthesis of compounds of formula (II), equally starting from a functionalized dimethylmaleic acid as one reactant which is reacted with a malonic acid derivative can be illustrated as follows. Groups corresponding to A¹ to A³ or R¹ to R³ or R'¹ to R'², in the target compound may be further converted to other groups A¹ to A³ or R¹ to R³ or R'¹ to R'² as desired/necessary during the course of the reaction sequence:

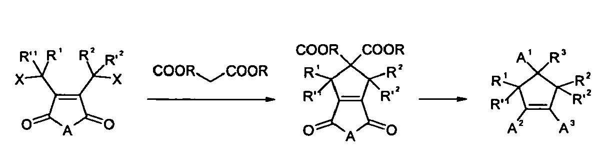

In the formulae, R represents hydrogen, alkyl or aryl, preferably hydrogen or alkyl, A is oxygen or NRi, Ri represents hydrogen, alkyl or aryl, preferably alkyl or aryl and X represents a leaving group, such as bromine, chlorine, iodine, mesylate, triflate or tosylate and R¹, R², R³, R'¹, R'², A¹, A² and A³ are as defined for formula (I). A similar approach was described in a different context in Journal of Organic Chemistry, 65(11), 2000, 3520-3524*.*

Yet another alternative that could be contemplated to arrive at the compounds of formula (II) is illustrated in the following scheme. Groups corresponding to A¹ to A³ or R¹ to R³ or R'¹ to R'² in the target compound may be further converted to other groups A¹ to A³ or R¹ to R³ or R'¹ to R'² as desired/necessary during the course of the reaction sequence:

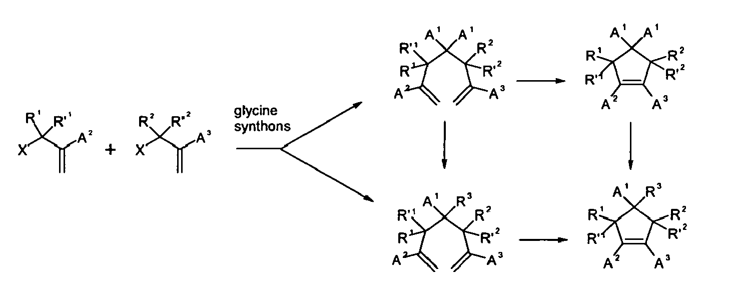

In the formulae, X represents a leaving group, such as bromine, chlorine, iodine, mesylate, triflate or tosylate and R¹, R², R³, R'¹, R'², A¹, A² and A³ are as defined for formula (I).
In this route, the cyclopentene cycle is obtained by an olefin metathesis reaction.

Yet another alternative that could be contemplated to arrive at the compounds of formula (II) is illustrated in the following scheme. Groups corresponding to A¹ to A³ or R¹ to R³ or R'¹ to R'² in the target compound may be further converted to other groups A¹ to A³ or R¹ to R³ or R'¹ to R'² as desired/necessary during the course of the reaction sequence:

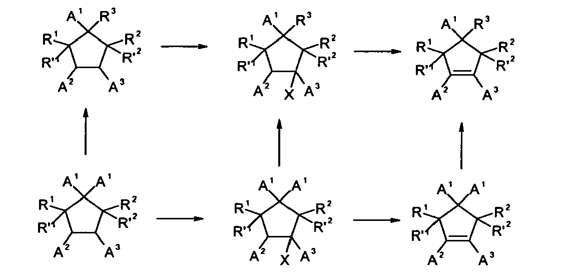

In the formulae, X represents a leaving group, such as bromine, chlorine, iodine, mesylate, triflate or tosylate and R¹, R², R³, R'¹, R'², A¹, A² and A³ are as defined for formula (I).
In this route, the cyclopentene cycle is obtained through elimination of HX from the corresponding intermediate bearing an appropriate leaving group.

In a further embodiment, the invention also concerns a method for the preparation of a compound of formula (VII) 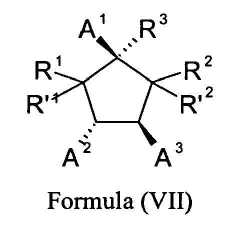 wherein A¹, A², A³, R¹, R'¹, R², R'² and R³ are defined as for formula (I). According to the present invention, compounds of formula (VII) can be prepared by treatment of a compound of formula (I) obtained according to the methods disclosed above with a base such as sodium hydroxide or potassium hydroxide or an alkali metal salt of a C1-C4 alkyl-OH such as sodium ethanolate or potassium ethanolate.

### EXAMPLES

All reagents were commercial grade and used without further purification. Commercially available anhydrous solvents were used for reactions conducted under inert atmosphere. Silica gel generally used for column chromatography was SDS silica gel (60AAC 40-63 µM). Thin layer chromatography was carried out using pre-coated silica gel F-254plate. ¹H NMR spectra were recorded on a Bruker 300 or 400 MHz spectrometer. Proton chemical shifts are listed relative to residual CDCl₃ (7.27 ppm) or D₂O (4.60 ppm). Splitting patterns are designated as s (singlet), d (doublet), dd (double-doublet), t (triplet), q (quartet), m (multiplet), br (broad).
Electrospray MS spectra were obtained on a Waters micromass platform LCMS spectrometer.
All mass spectra were full-scan experiments (mass range 100-800 amu). Mass spectra were obtained using an electro spray ionization. The HPLC system was a Waters platform with a 2767 sample manager, an 2525 pump, a photodiode array detector (190-400 nM). The column used was an Xterra C₁₈ 3.5 µM (4.6 x 50 mm) in analytical mode and an Xterra C18 OBD 5 µM (30 x 100 mm) in preparative mode. The mobile phase in both cases consisted in an appropriate gradient of A and B. A was water with 0.05 % of TFA and B was acetonitrile with 0.05 % of TFA. Flow rate was 1 ml/minute in analytical mode and 25 ml/minute in preparative mode. All LCMS were performed at room temperature.
Condition in preparative mode: 95 % A for 5 minutes, then from 95 % A to 80 % A in 10 minutes.

### Example 1: synthesis of (1S, 3R, 4S) -1-aminocyclopentane-1,3,4-tricarboxylic acid (ACPT-1)

### Step one:

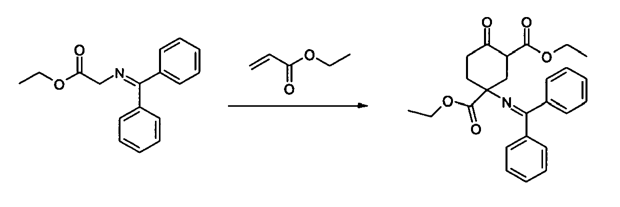

To a stirred solution of Bu₄NBr (3.0 g) and sodium ethoxide (31.8 g) in acetonitrile (200 mL) cooled by an ice bath, under a nitrogen atmosphere, a solution of ethyl N-(diphenylmethylene)glycinate (25.0 g) and ethyl acrylate (51.0 g) in acetonitrile (200 mL) was added. The mixture was stirred at 0°C to 4°C overnight then 4 Hrs at 5°C to 10°C and then was filtered. The filtrate was hydrolyzed with 5% citric acid (aq.) and extracted with ethyl acetate. The organic layer was dried and solvent removed to give an orange oil which was purified by chromatography on silica gel (ethyl acetate : isohexane (10 :90)) to give a mixture of cyclohexanone diastereomers as a yellow oil (30.0 g, 76%).
M/Z (M+H₂O-benzophenone+H)⁺ = 258.

### Step two:

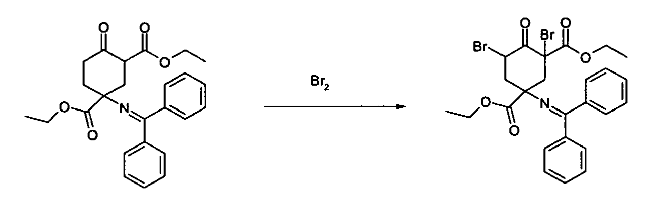

To a solution of cyclohexanone (29.2 g) in CHCl₃ (150 mL), cooled to 0°C, bromine (1 M in CHCl₃ - 138.5 mL) was slowly added over 20 minutes. The reaction mixture was stirred at 0°C for 1.5 Hrs, then was allowed to warm to room temperature overnight. The solution was poured onto a mixture of ice and saturated sodium bicarbonate (aq.) then was extracted with DCM. The organic layer was washed with brine, dried over MgSO₄ and then evaporated to give a yellow oil which was purified by chromatography on silica gel (ethyl acetate : isohexane (20 : 80)) to give the dibromo derivative (28.0 g, 70%).
M/Z (M+H)⁺ = 580.

### Step three:

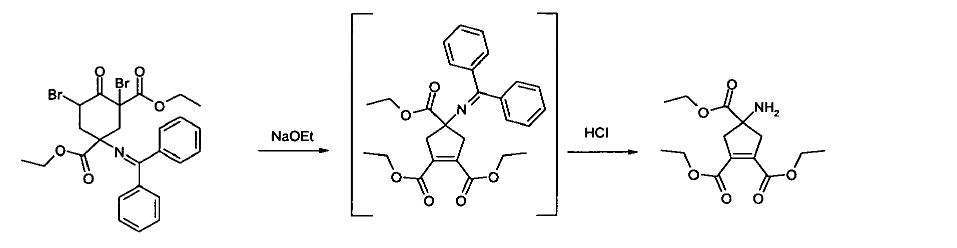

To a solution of sodium ethoxyde (6.7 g) in dry ethanol (300 mL) at 0°C, the dibromo material (26.0 g) in solution in dry ethanol (300 mL) was added. The reaction mixture was stirred at 0°C for 1 Hrs, then was poured into aqueous 1M HCl (150 mL) at 0°C. The reaction mixture turned cloudy, and was stirred 30 minutes below 10°C. The clear solution was extracted with diethyl ether. The aqueous layer was neutralized with saturated NaHCO₃ and extracted with ethyl acetate. The organic layer was dried over Na₂SO₄, concentrated and purified by chromatography on silica gel (ethyl acetate : isohexane (60 :40)) to give the cyclopentene amino-ester (2.9 g, 22%).
¹H-NMR (300 MHz, CDCl₃): 1.28 (m, 9H), 2.66 (d, 2H, J=15.4 Hz), 3.35 (d, 2H, J=15.4 Hz), 4.22 (m, 6H).

### Step four:

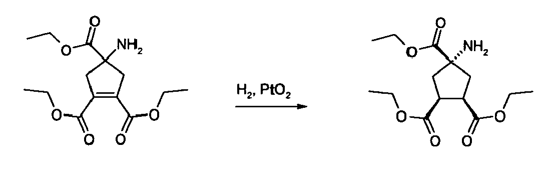

To a solution of cyclopentene derivative (1.5 g) in ethanol (90 mL), PtO₂ (60 mg) was added. The reaction mixture was purged with hydrogen, and a pressure of 1 atm of hydrogen was maintained overnight. The suspension was filtered and the filtrate was concentrated to give 1-aminocyclopentane-1,3,4-tricarboxylic acid triethyl ester as a light brown oil (1.5 g, 99%).
¹H-NMR (300 MHz, CDCl₃): 1.24 (m, 9H), 1.95 (m, 2H), 2.54 (m, 2H), 3.46 (m, 2H), 4.15 (m, 6H).

### Step five:

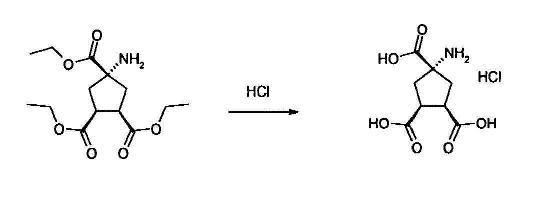

A solution of amino-ester (25 mg) in a mixture of dioxane and aqueous 6M HCl (1:1, 2mL) was stirred at 40°C for 7 days. Reaction mixture concentration gives the hydrochloric acid salt of ACPT-I.
¹H-NMR (400 MHz, D₂O): 2.33-2.41 (m, 2H), 2.75-2.84 (m, 2H), 3.45-3.52 (m, 2H).

### Example 2: synthesis of (IS, 3R, 4S)-1-{[(2'S)-2'-amino-4'-methylpentanoyl]amino}cyclopentane-1,3,4-tricarboxylic acid (L-Leu-ACPT-I)

The material isolated from step 4 of example 1 was used to generate example 2.

### Step one:

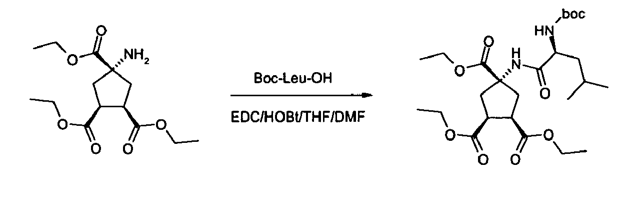

To a solution of 1-Aminocyclopentane-1,3,4-tricarboxylic acid triethyl ester (920 mg), Boc-Leu-OH (774 mg) and HOBt (452 mg) in THF/DMF, cooled to 0°C, EDC (641 mg) was added portionwise. The reaction mixture was allowed to warm to room temperature overnight. The reaction mixture was hydrolyzed with a cold citric acid solution and was extracted with ethyl acetate. The organic layer was washed with saturated NaHCO₃, with brine, was dried over MgSO₄, then was concentrated. The crude product was purified by chromatography on silica gel (ethyl acetate : hexane (1 : 3)) to give the protected L-Leu-ACPT I as a yellow oil (1.4 g, 89 %).
¹H-NMR (300 MHz, CDCl₃): 0.94 (dd, 6H, J=6.3, 5.9 Hz), 1.24 (m, 9H), 1.45 (s, 9H), 1.65 (m, 3H), 2.41 (m, 2H), 2.71 (m, 2H), 3.39 (m, 2H), 4.01 (m, 1H), 4.14 (m, 6H), 4.82 (brd, 1H, J=7.9 Hz), 6.86 (bs, 1H).

### Step two:

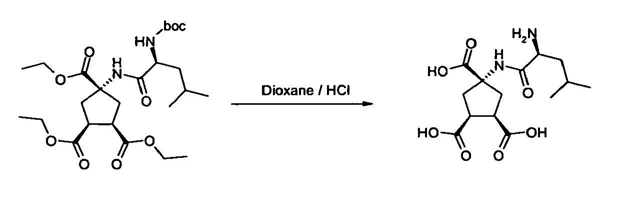

The protected L-Leu-ACPT I (373 mg) in solution in dioxane : 2M HCl mixture (1:1 ratio - 7.3 mL) was heated at 80°C for 24 Hrs. The reaction mixture was cooled, solvent volume was reduced to a minimum and the product was isolated using prep LCMS to afford the trifluoroacetic acid salt of the L-Leu-ACPT I as a white solid (120 mg, 50%). ¹H-NMR (400 MHz, D₂O): 0.89 (dd, 6H, J=6.4, 6.4 Hz), 1.65 (m, 3H), 2.38 (m, 2H), 2.62 (m, 2H), 3.34 (m, 2H), 3.93 (m, 1H).
M/Z (M+H)⁺= 331.

## Claims

1. A method for the preparation of a compound of formula (I) 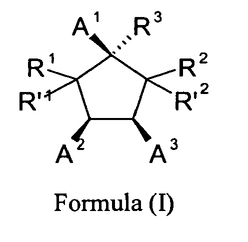 wherein
A¹, A² and A³ are independently selected from a carboxylic acid group and a salt, ester or amide thereof, from an aldehyde group and protected forms thereof, from a -COCCl₃ group and protected forms thereof, from a -COCBr₃ group and protected forms thereof, and from a -CH₂-OH group and protected forms thereof;
R¹, R'¹, R² and R'² are independently selected from hydrogen, optionally substituted alkyl, optionally substituted aryl, halogen, OH or O-alkyl;
R³ is selected from an amino group which may be mono- or disubstituted, an isocyano group, an imino group which may be substituted, a nitro group and a -NHY group, with Y representing a -(W)ₙ-H group wherein W is an amino-acyl group corresponding to a natural or non-natural amino-acid and n is an integer comprised between 1 and 10;
and wherein A¹ and R³, together with the carbon atom to which they are attached, may form a cyclic group;
said method comprising the step of hydrogenating compound of formula (II): 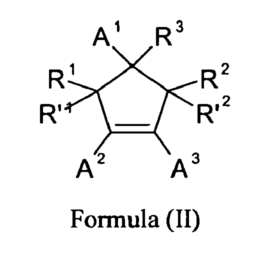 wherein A¹, A², A³, R¹, R'¹, R², R'² and R³ are defined as for formula (I), in the presence of a catalyst.

2. The method of claim 1, wherein A¹, A² and A³ in formula (I) are carboxylic acid groups.

3. The method of claim 2, wherein A¹, A² and A³ in formula (II) are ester groups, and wherein the process further comprises the step of deprotecting these esters into the carboxylic acid groups.

4. The method of any of claims 1 to 3, wherein R³ is selected from NH₂ or -NHY.

5. The method of any of claims 1 to 4, wherein the hydrogenation catalyst is selected from heterogeneous catalysts based on palladium, rhodium, ruthenium, nickel, platinum or iridium or homogeneous Wilkinson's or Crabtree's catalysts.

6. The method according to claim 5, wherein the hydrogenation catalyst is PtO₂.

7. The method according to claim 5, wherein the hydrogenation catalyst is Pd/C.

8. The method according to any of claims 1 to 7 above, further comprising the step of subjecting a compound of formula (III) : 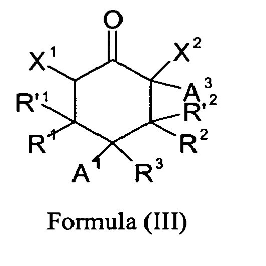 wherein
A¹, A³, R¹, R², R'¹, R'² and R³ are as defined in any of claims 1 to 3 and X¹ and X² may be the same or different and represent each a leaving group,
to a Favorskii rearrangement to yield a compound of formula (II).

9. A method for the preparation of a compound of formula (VII) 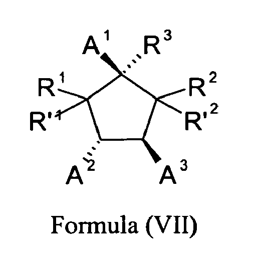 wherein A¹, A², A³, R¹, R'¹, R², R'² and R³ are defined as for formula (I), comprising synthesis of a compound of formula (I) according to the method of any of claims 1 to 7 above, and further comprising the treatment of the resulting compound of formula (I) with a base.
